(19) Europäisches Patentamt
European Patent Office
Office européen des brevets

(11) Numéro de publication : **0 015 038**
**B1**

(12) **FASCICULE DE BREVET EUROPÉEN**

(45) Date de publication du fascicule du brevet : **19.12.84**

(51) Int. Cl.³ : **A 61 K 31/415**, C 07 D233/72

(21) Numéro de dépôt : **80200137.0**

(22) Date de dépôt : **18.02.80**

(54) **Composition pharmaceutique comprenant un dérivé de diphénylhydantoïne, dérivés mis en oeuvre et leur préparation.**

(30) Priorité : **20.02.79 FR 7904209**

(43) Date de publication de la demande : **03.09.80 Bulletin 80/18**

(45) Mention de la délivrance du brevet : **19.12.84 Bulletin 84/51**

(84) Etats contractants désignés : **AT BE CH DE GB IT LU NL SE**

(56) Documents cités :
**AUSTRALIAN JOURNAL OF CHEMISTRY, vol. 31, no. 2, février 1978, Melbourne, AU R.G. COOMBE et al. "Preparation of 5,5-bis(2-deuterophenyl) hydantoin", pages 451-453**
**JOURNAL OF LABELLED COMPOUNDS AND RADIO-PHARMACEUTICALS, vol. 13, no. 4, 1977, Chichester (GB) D.L. CASEY et al. "A convenient synthesis of deuterium labelled diphenylhydantoin", pages 623-625**
**CHEMICAL ABSTRACTS, vol. 84, no. 9, 1 mars 1976, page 509, abrégé 59303p. Columbus, Ohio, USA J.A. KEPLER "Preparation of tritium labeled diphenylhydantoin of high specific activity"**

(73) Titulaire : **REGION WALLONNE représentée par le Ministre des Technologies nouvelles de la région wallonne**
**Avenue des Arts, 19 H**
**B-1040 Bruxelles (BE)**

(72) Inventeur : **Dumont, Pierre**
**13, avenue Moine Olbert**
**B-5800 Gembloux (BE)**
Inventeur : **Poupaert, Jacques**
**Rue du Palier, 22**
**B-1348 Ottignies (BE)**

(74) Mandataire : **Blanchart, André et al**
**Cellule de Gestion des Contrats Technologiques**
**Place Joséphine Charlotte 3 (Boîtes 27, 28)**
**B-5100 Namur (BE)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

EP 0 015 038 B1

**Description**

La présente invention est relative à une composition pharmaceutique, en particulier psychotrope, contenant comme substance active un dérivé de phénylhydantoïne.

On connaît actuellement des compositions pharmaceutiques à action antiépileptique comprenant, à titre de substance active, de la 5,5-diphénylhydantoïne. Il est apparu que la 5,5-diphénylhydantoïne présente des inconvénients en ce qui concerne son absorption, sa distribution, son métabolisme et son excrétion.

Afin d'étudier ces phénomènes sur la 5,5-diphényldantoïne, il a déjà été proposé de marquer cette substance par du deutérium pour la réalisation des analyses. On a déjà préparé dans ce but de la 5,5-diphénylhydantoïne pentadeutériée et décadeutériée. Ces substances n'ont cependant jamais été utilisées que dans un but d'analyse biochimique.

La présente invention a pour but de mettre au point une composition pharmaceutique psychotrope qui améliore l'activité anticonvulsivante obtenue par la 5,5-diphénylhydantoïne, et qui ne présente pas les inconvénients de cette dernière substance.

On a découvert qu'on peut résoudre ce problème suivant l'invention par une composition pharmaceutique comprenant comme constituant actif, une diphénylhydantoïne deutériée répondant à la formule générale

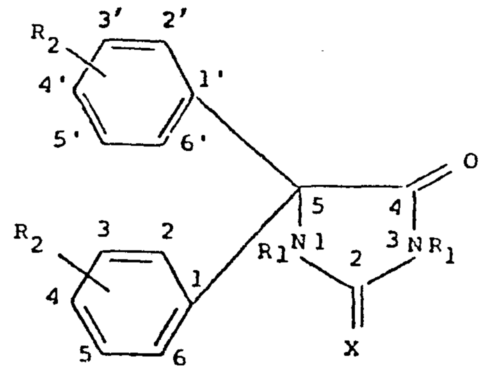

dans laquelle un des groupes phényle est monodeutérié, les radicaux $R_1$ sont identiques ou différents et représentent un atome d'hydrogène ou un groupe méthyle, $R_2$ représente un atome d'hydrogène ou d'halogène ou un radical alkyle, linéaire ou ramifié, comprenant de 1 à 4 atomes de carbone, un radical alcoxy, linéaire ou ramifié, comprenant de 1 à 4 atomes de carbone, un radical trifluorométhyle, hydroxy, nitro ou amino, X représente un groupement oxo, thio ou méthylène, un de ses énantiomères, ou un de ses sels avec une base physiologiquement acceptable, en combinaison avec un véhicule pharmaceutiquement acceptable.

Les formules des énantiomères de la formule générale sont les suivantes

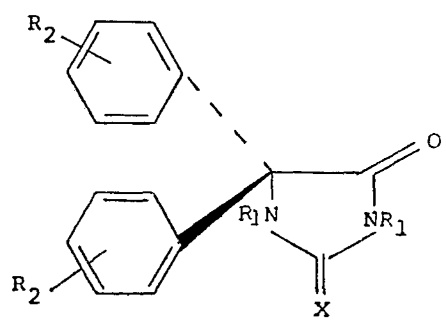

et

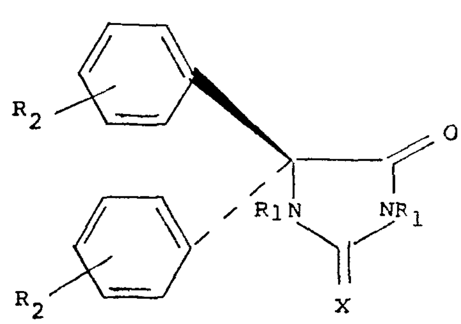

On a de plus mis au point un nouveau dérivé monodeutérié de la phénylhydantoïne, ce composé chimique et son procédé de préparation faisant par conséquent également l'objet de la présente invention. Il s'agit de la (R,S)-5-(paradeutérophényl)-5-phényldantoïne et de ses énantiomères.

La préparation des substances actives suivant l'invention est décrite de manière plus détaillée à l'aide des exemples ci-après, sans être pour autant limitée par ces derniers.

Exemple 1

Procédé de préparation de 5-(paradeutérophényl)-5-phénylhydantoïne

a) Dans une bombe en acier de 500 ml, on introduit 60 g de 4-bromo benzophénone en solution dans 300 ml de diméthylformamide, 100 g de carbonate d'ammonium solide et 30 g de cyanure de potassium en solution dans 100 ml d'eau. Après fermeture de la bombe, le mélange réactionnel est chauffé à 120 °C durant 5 jours, puis déversé sous agitation mécanique rapide dans 2 l d'eau à 80 °C, acidifié (HCl concentré) et essoré après refroidissement au bain de glace. On obtient un produit brut, qui est traité par de la soude 2 N (500 ml). La suspension est filtrée ; le filtrat est extrait par trois fois à l'éther (300 ml au total). La phase aqueuse est acifiée à l'acide chlorhydrique concentré pour donner 59 g de 5-(parabromophényl)-5-phénylhydantoïne, sous forme de produit brut. La recristallisation dans l'éthanol fournit 40 g de produit pur.

P. F. : 237-239 °C.
CCM : Rf 0,42 ($CHCl_3$ : acétone 90 : 10, v/v).
IR : 1 775, 1 715 $cm^{-1}$ ($\upsilon c = o$) (KBr)

b) Dans une bouteille à hydrogéner (Parr) de 500 ml, on introduit 30 g de 5-(parabromophényl)-5-phénylhydantoïne que l'on dissout dans 250 ml de dioxanne anhydre et 30 ml de triéthylamine. On ajoute 5 g de palladium sur charbon à 10 %. La suspension est agitée durant 24 h sous une pression initiale de 0,344 75 MPa de deutérium.

Le catalyseur et le chlorhydrate de triéthylamine sont filtrés et on évapore le filtrat à sec sous vide. La recristallisation dans l'éthanol fournit 21 g du produit cité en rubrique.

P. F. : 297-298 °C.
CCM : Rf 0,56 ($CHCl_3$ : acétone, 90 : 10, v/v)
IR : 1 775, 1 740, 1 720 $cm^{-1}$ ($\upsilon c = o$) KBr
SM : 253 (m+), 224, 209, 181, 166, 105, 104, 78, 77
Analyse élémentaire :

| | | | |
|---|---|---|---|
| Calculé : | N 11,06 | C 71,12 | H 5,17 |
| Trouvé : | N 10,89 | C 71,41 | H 4,93 |

Exemple 2

Procédé de préparation de 5-(paradeutérophényl)-5-phénylhydantoïne

a) Dans un ballon de 250 ml muni d'un réfrigérant, on introduit 3 g de parachlorobenzile et 80 ml d'éthanol (95 %). On dissout le produit par chauffage à reflux durant 5 minutes. On ajoute ensuite 1,5 g d'urée et 1,2 g de KOH solide, ainsi que 20 ml d'éthanol supplémentaires.

Un précipité blanc apparaît immédiatement. On chauffe à reflux durant 8 h. On déverse alors le mélange réactionnel dans 400 ml d'eau, on filtre, on acidifie le filtrat (HCl concentré). On essore la 5-(parachlorophényl)-5-phénylhydantoïne obtenue. On la sèche sous vide (80 °C/30 mmHg) et on la recristallise dans l'éthanol : eau (80 : 20, v/v). Production : 0,85 g.

P. F : 243-244 °C (littérature : 243 °C).
CCM Rf : 0,45 ($CHCl_3$ : acétone, 90 : 10, v/v)

b) On procède de manière analogue à l'exemple 1b.

Exemple 3

Procédé de préparation de 5-phényl-$d_5$-5-phénylhydantoïne.

a) Dans un ballon à trois cols muni d'une ampoule à additionner, d'un agitateur mécanique et d'un réfrigérant surmonté d'un tube à chlorure calcique, on introduit 100 ml de benzène-$D_6$, 60 g de trichlorure d'aluminium anhydre et 200 ml de sulfure de carbone. Ce mélange est refroidi au bain de glace durant 15 minutes sous agitation mécanique. En 20 minutes, on ajoute alors goutte à goutte et sous agitation rapide 55 ml de chlorure de benzoyle dilué dans 100 ml de sulfure de carbone. On maintient encore durant 30 minutes le mélange réactionnel dans le bain de glace après la fin de l'addition, puis on chauffe à reflux durant 6 h. On décompose le mélange réactionnel sur 2 kg de glace, on extrait ensuite au dichlorométhane. On évapore à sec. Le résidu est repris dans 300 ml de benzène et traité par 300 ml de NaOH 2 N. On sépare les phases et on extrait la phase aqueuse par 2 × 100 ml de benzène. La phase organique est séchée ($MgSO_4$) et concentrée sous vide. On distille ensuite sous vide, ce qui donne 71 g de benzophénone-$d_5$. $Eb_{40}$dmdmdHdg : 190-195 °C.

b) Dans une bombe en acier de 500 ml, on introduit successivement 60 g de benzophénone-$d_5$, 300 ml de diméthylformamide, 30 g de cyanure de potassium dissous dans 100 ml d'eau et 100 g de carbonate d'ammonium. Le mélange réactionnel est chauffé durant 5 jours à 120 °C dans un bain d'huile thermostatisé.

L'isolement du produit cité en rubrique se fait comme précédemment pour la 5-(parabromophényl)-5-phénylhydantoïne.

La production de produit brut est de 58,5 g. La production de produit recristallisé dans l'éthanol est de 44 g.

P. E. : 297-298 °C.

CCM : Rf 0,56 ($CHCl_3$ : acétone, 90 : 10, v/v).

IR : 1 775, 1 740, 1 720 $cm^{-1}$ (υc = o) KBr.

SM : 257 (m+), 228, 214, 184, 170, 109, 104, 82, 77

Analyse élémentaire :

| | | |
|---|---|---|
| Calculé : | N 10,86 | C 69,85 |
| Trouvé : | N 10,72 | C 70,29 |

Les compositions suivant l'invention peuvent être utilisées à titre de produit psychotrope, en particulier anticonvulsivant, et donc être administrées en particulier lors du traitement de l'épilepsie.

On peut administrer ces compositions sous la forme de comprimés, de cachets, de gélules, de capsules, de dragées, ou sous la forme de solutions. On peut, par exemple les administrer par voie orale sous la forme de gélules contenant une dose unitaire de 30 à 200 mg de substance active en poudre, la dose journalière étant de 1,5 à 5 mg/kg de poids de corps. On peut aussi administrer les compositions suivant l'invention, par exemple par voie intrapéritonéale, en solution, ou par voie intraveineuse, par dose unitaire de 100 à 200 mg de substance active en solution.

On a soumis les substances actives suivant l'invention à des essais en vue d'examiner leur activité et leurs effets en comparaison de la diphénylhydantoïne connue (DPH). Dans la suite de ces essais, on désignera le 5-(paradeutérophényl)-5-phénylhydantoïne par DPH-$d_1$ et la 5-phényl-$d_5$-5-phénylhydantoïne par DPH-$d_5$.

Pour examiner l'activité anticonvulsivante de ces substances, on les a soumises à l'essai de protection contre les convulsions provoquées par du pentylène tétrazole chez le rat femelle adulte (Souche Wistar : 200-220 g). Une, deux ou quatre heures après l'administration orale des hydantoïnes (100 et 200 mg/kg : solution dans du diméthylsulfoxyde/propylèneglycol), les animaux reçoivent une dose de 100 mg/kg de pentylène tétrazole (PTZ) par voie intrapéritonéale puis ils sont tenus en observation durant 1 h. La non-apparition de convulsions est prise comme une réponse positive. Les résultats sont exprimés dans le Tableau I ci-dessous sous la forme du rapport : nombre d'animaux protégés/nombre total d'animaux.

Tableau I

Protection des rats contre l'effet convulsivant du PTZ

| | DPH | DPH-$d_1$ | DPH-$d_5$ |
|---|---|---|---|
| A. Doses de substances actives à temps fixe (1 h) | | | |
| 100 mg/kg | 4/25 | 9/14 | 7/20 |
| 200 mg/kg | 5/35 | 11/30 | 11/35 |
| B. Temps à dose fixe (100 mg/kg). | | | |
| 1 h | 4/25 | 9/14 | 7/20 |
| 2 h | 2/10 | 4/10 | 2/10 |
| 4 h | 0/10 | 2/10 | 0/10 |

L'analyse de ces résultats (Tableau II) a été faite par une méthode non paramétrique (Tables de fréquence 2 × 2 : $X^2$ différences). La recherche de différences significatives a porté sur les aspects suivants : DPH et DPH deutériées, à 1 h aux doses de 100 et 200 mg/kg, DPH et DPH deutériées à tous temps, à la dose de 100 mg/kg, entre les doses 100 et 200 mg/kg, entre les temps.

Tableau II

Analyse statistique

Méthode non paramétrique, Tables de fréquence

2 × 2 ; $X^2$ différences

| | DPH | DPH-$d_1$ | DPH-$d_5$ |
|---|---|---|---|
| 1. Entre DPH et DPH deutériées (à 1 h) | | | |
| 100 mg/kg | | 9,42** | 2,17 |
| 200 mg/kg | | 4,36* | 2,92 |
| 2. Entre DPH et DPH deutériées à tous temps | | | |
| 100 mg/kg | | 9,40** | 1,22 |

Tableau II (suite)

| | DPH | DPH-$d_1$ | DPH-$d_5$ |
|---|---|---|---|
| 3. Entre les doses (100 et 200 mg/kg) | NS | NS | NS |
| 4. Entre les temps $\frac{1\ h}{2\ h + 4\ h}$ | NS | 3,93* | 3,58 |

* $p < 0{,}05$
** $p < 0{,}01$
NS : non significatif.

L'analyse statistique indique clairement que la DPH-$d_1$ protège davantage les animaux contre l'effet convulsivant du PTZ que la DPH, les différences sont hautement significatives à 100 mg/kg (9,42**) et significatives à 200 mg/kg (4,36*). Cet effet se maintient pendant 4 heures (9,40**) mais est plus marqué à la première heure (3,93*). Une différence allant dans le même sens, proche de la limite de signification, est également observée avec la DPH-$d_5$.

Des déterminations de taux sanguins ont été effectuées sur rats et souris, de l'un et l'autre sexe, après administration des substances à examiner par voie intraveineuse et on a utilisé pour ce faire, comme méthode de dosage, la mesure de la radioactivité des produits marqués au $^{14}C$.

Après injection par voie intraveineuse, sur 5 rats femelles, d'une dose de 50 mg/kg de substance à examiner (solution alcaline), on examine, en utilisant la méthode de marquage au $^{14}C$, l'évolution de la concentration sanguine (µg/ml) en la substance, en fonction du temps. (Voir figure unique annexée.)

Si l'on fixe son attention sur le comportement comparatif de la DPH d'une part et de ses analogues deutériés de l'autre, des différences nettes apparaissent et cela dans les diverses expériences entreprises.

Les teneurs en DPH, DPH-$d_1$ et DPH-$d_5$ des différents tissus ont été déterminées à deux temps après administration par voie orale (souris mâles, rats femelles, dosage par chromatographie en phase gazeuse).

L'ensemble des résultats est consigné dans le tableau III ci-dessous. Les valeurs indiquées représentent les rapports des concentrations des substances actives dans le cerveau et le sang.

Après administration par voie orale, ces valeurs, tant chez le rat que la souris manifestent une tendance, marquée à très marquée, à être supérieures pour les DPH deutériées par rapport à la DPH elle-même. La deutériation modifierait donc la répartition cerveau/sang en faveur du cerveau, phénomène qui serait en accord avec la plus grande activité anticonvulsivante de la DPH-$d_1$ et de la DPH-$d_5$.

Tableau III
Répartition tissulaire
Administration par voie orale
Dosage par chromatographie en phase gazeuse

Souris 20-25 g ♂ 100 mg/kg

| | Après 2 heures | | Après 24 heures | |
|---|---|---|---|---|
| | DPH | DPH-$d_5$ | DPH | DPH-$d_5$ |
| Rapport cerveau/sang | 1,47 ± 0,10 | 2,35 ± 0,26 | 2,09 ± 0,22 | 2,97 ± 0,30 |

Rat : ± 200 g ♀ 100 mg/kg

| | Après 1 heure | | |
|---|---|---|---|
| | DPH | DPH-$d_5$ | DPH-$d_1$ |
| Rapport cerveau/sang | 0,90 ± 013 | 2,15 ± 0,37 | 2,10 ± 0,41 |
| | **Après 2 heures** | | |
| | DPH | DPH-$d_5$ | DPH-$d_1$ |
| Rapport/cerveau sang | 5,91 ± 0,93 | 4,49 ± 1,33 | 1,55 ± 3,7 |

Il ressort nettement de tous ces essais que la deutériation de la diphénylhydantoïne améliore l'activité anticonvulsivante du produit, modifie sa pharmacocinétique sanguine et favorise l'accumulation du produit dans le tissu cérébral.

**Revendications**

1. Composition pharmaceutique, en particulier psychotrope, caractérisée en ce qu'elle comprend, comme constituant actif, une diphénylhydantoïne deutériée répondant à la formule générale

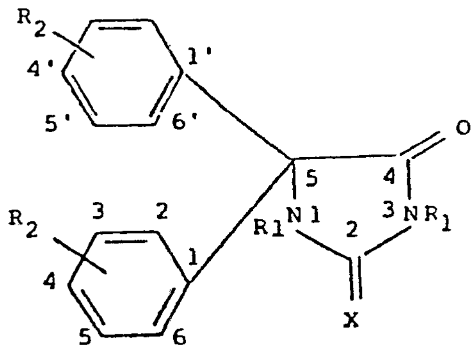

dans laquelle un des groupes phényle est monodeutérié, les radicaux $R_1$ sont identiques ou différents et représentent un atome d'hydrogène ou un groupe méthyle, $R_2$ représente un atome d'hydrogène ou d'halogène ou un radical alkyle, linéaire ou ramifié, comprenant de 1 à 4 atomes de carbone, un radical alcoxy, linéaire ou ramifié, comprenant de 1 à 4 atomes de carbone, un radical trifluorométhyle, hydroxy, nitro ou amino, X représente un groupement oxo, thio ou méthylène, un de ses énantiomères, ou un de ses sels avec une base physiologiquement acceptable, en combinaison avec un véhicule pharmaceutique acceptable.

2. Composition suivant la revendication 1, caractérisée en ce que, dans la formule générale lorsque $R_2$ représente un atome d'halogène, celui-ci est un groupe chloro, fluoro, bromo ou iodo.

3. Composition suivant l'une ou l'autre des revendications 2 et 3, caractérisée en ce que la diphénylhydantoïne deutériée est choisie dans le groupe formé par la (R,S)-5-(paradeutérophényl)-5-phénylhydantoïne, la (R)-5-(paradeutérophényl)-5-phénylhydantoïne et la (S)-5-(paradeutérophényl)-5-phénylhydantoïne.

4. Diphénylhydantoïne deutériée répondant à la formule générale, caractérisée en ce qu'elle est constituée par la (R,S)-5-(paradeutérophényl)-5-phénylhydantoïne, la (R)-5-(paradeutérophényl)-5-phénylhydantoïne ou la (S)-5-(paradeutérophényl)-5-phénylhydantoïne.

5. Procédé de préparation d'une 5-(paradeutérophényl)-5-phénylhydantoïne répondant à la formule générale, caractérisé en ce que l'on fait réagir du parachlorobenzile en présence d'hydroxyde de potassium et d'urée de manière à obtenir de la 5-(parachlorophényl)-5-phénylhydantoïne, et en ce que l'on soumet la 5-(parachlorophényl)-5-phénylhydantoïne à une deutériation en présence de palladium sur charbon.

6. Procédé de préparation d'une 5-(paradeutérophényl)-5-phénylhydantoïne répondant à la formule générale, caractérisé en ce que l'on fait réagir de la parabromobenzophénone en présence de diméthylformamide, de carbonate ammonique, de cyanure de potassium et d'eau de manière à obtenir de la 5-(parabromophényl)-5-phényl hydantoïne et en ce que l'on soumet la 5-(parabromophényl)-5-phénylhydantoïne à une deutériation en présence de palladium sur charbon.

7. Composition suivant l'une quelconque des revendications 1 à 3, caractérisée en ce qu'elle est présentée sous forme de dose unitaire.

8. Composition suivant la revendication 7, caractérisé en ce qu'elle est présentée sous forme de comprimé, de cachet, de capsule, de dragée ou de solution.

9. Procédé de préparation d'une composition pharmaceutique psychotrope suivant l'une quelconque des revendications 1 à 3 et 7 et 8, caractérisé en ce que l'on mélange une diphénylhydantoïne deutériée de formule générale avec des véhicules et/ou excipients solides ou liquides, inertes, non toxiques, utilisés en pharmacie galénique et en ce qu'on donne au mélange une forme appropriée à son administration telle que celle de comprimés, de cachets, de capsules, de dragées ou de solutions buvables ou injectables et autres.

**Claims**

1. A pharmaceutical composition, in particular psychotropic, characterised in that it comprises, as the active constituent, a deuterated diphenylhydantoin of the general formula

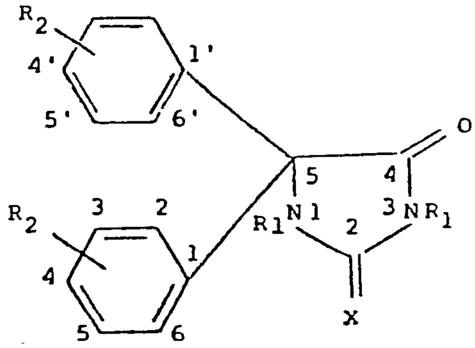

wherein one of the phenyl groups is monodeuterated, the radicals $R_1$ are identical or different and represent a hydrogen atom or a methyl group, $R_2$ represents a hydrogen or halogen atom or an alkyl radical, linear or branched, comprising 1 to 4 carbon atoms, an alkoxy radical, linear or branched, comprising 1 to 4 carbon atoms, a trifluoromethyl, hydroxy, nitro or amino radical, X represents an oxo, thio or methylene grouping, one of its enantiomers, or one its salts with a physiologically acceptable base, in combination with an acceptable pharmaceutical vehicle.

2. A composition according to Claim 1, characterised in that, in the general formula, when $R_2$ represents a halogen atom it is a chloro, fluoro, bromo or iodo group.

3. A composition according to any one of claims 1 and 2, characterised in that the deuterated diphenylhydantoin is selected from the group comprising (R,S)-5-(paradeuterophenyl)-5-phenylhydantoin, (R)-5-(paradeuterophenyl)-5-phenylhydantoin, and (S)-5-(paradeuterophenyl)-5-phenylhydantoin.

4. A deuterated diphenylhydantoin of the general formula, characterised in that it is constituted by (R,S)-5-(paradeuterophenyl)-5-phenylhydantoin, (R)-5-(paradeuterophenyl)-5-phenylhydantoin or (S)-5-(paradeuterophenyl)-5-phenylhydantoin.

5. A method for the preparation of a 5-(paradeuterophenyl)-5-phenylhydantoin of the general formula, characterised in that parachlorobenzil is reacted in the presence of potassium hydroxide and urea so as to obtain 5-(parachlorophenyl)-5-phenylhydantoin, and in that the 5-(parachlorophenyl)-5-phenylhydantoin is subjected to deuteration in the presence of palladium on carbon.

6. A method for the preparation of a 5-(paradeuterophenyl)-5-phenylhydantoin of the general formula, characterised in that parabromobenzophenone is reacted in the presence of dimethylformamide, ammonium carbonate, potassium cyanide and water so as to obtain 5-(parabromophenyl)-5-phenylhydantoin, and in that the 5-(parabromophenyl)-5-phenylhydantoin is subjected to deuteration in the presence of palladium on carbon.

7. A composition according to any one of Claims 1 to 3, characterised in that it is provided in the form of a unit dose.

8. A composition according to Claim 7, characterised in that it is provided in the form of a tablet, cachet, capsule, dragee or solution.

9. A method for the preparation of a pharmaceutical psychotropic composition according to any one of Claims 1 to 3 and 7 and 8, characterised in that a deuterated diphenylhydantoin of the general formula is mixed with solid or liquid, inert, non-toxic vehicles and/or excipients used in galenical pharmacy, and in that the mixture is given a form appropriate to its administration, such as that of tablets, cachets, capsules, dragees or solutions which can be taken orally or injected and others.

**Ansprüche**

1. Pharmazeutische, insbesondere psychotrope Zusammensetzung, dadurch gekennzeichnet, daß sie als Wirkstoff ein deuteriertes Diphenylhydantoin der allgemeinen Formel

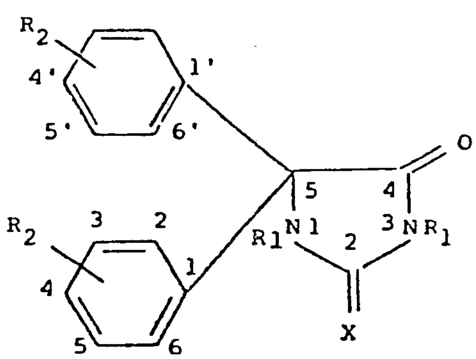

in welcher eine der Phenylgruppen monodeuteriert ist, die Reste $R_1$ gleich oder verschieden sein können und ein Wasserstoffatom oder eine Methylgruppe bedeuten, $R_2$ ein Wasserstoffatom oder ein Halogenatom oder ein linearer oder verzweigter Alkylrest mit 1 bis 4 Kohlenstoffatomen, ein linearer oder verzweigter Alkoxyrest mit 1 bis 4 Kohlenstoffatomen, ein Trifluormethylrest, Hydroxy, Nitro oder Amino ist, X eine Oxo-, Thio- oder Methylengruppe darstellt, enthält, oder eines der Enantiomeren einer solchen Verbindung oder ein Salz einer solchen Verbindung mit einer physiologisch annehmbaren Base, in Kombination mit einem pharmazeutisch annehmbaren Trägerstoff.

2. Zusammensetzung gemäß Anspruch 1, dadurch gekennzeichnet, daß, wenn in der allgemeinen Formel $R_2$ ein Halogenatom bedeutet, dieses Chlor, Fluor, Brom oder Jod ist.

3. Zusammensetzung gemäß einem der Ansprüche 1 und 2, dadurch gekennzeichnet, daß gekennzeichnet, daß das deuterierte Diphenylhydantoin ausgewählt ist aus der Gruppe gebildet von (R,S)-5-(Paradeuterophenyl)-5-phenylhydantoin, (R)-5-(Paradeuterophenyl)-5-phenylhydantoin und (S)-5-(Paradeuterophenyl)-5-phenylhydantoin.

4. Deuteriertes Diphenylhydantoin entsprechend der allgemeinen Formel, dadurch gekennzeichnet, daß es (R,S)-5-(Paradeuterophenyl)-5-phenylhydantoin, (R)-5-(Paradeuterophenyl)-5-phenylhydantoin oder (S)-5-(Paradeuterophenyl)-5-phenylhydantoin ist.

5. Verfahren zur Herstellung eines 5-(Paradeuterophenyl)-5-phenylhydantoins entsprechend der allgemeinen Formel, dadurch gekennzeichnet, daß man Parachlorbenzil in Anwesenheit von Kaliumhydroxid und Harnstoff in der Weise umsetzt, daß man 5-(Parachlorphenyl)-5-phenylhydantoin erhält, und daß man das 5-(Parachlorphenyl)-5-phenylhydantoin einer Deuterierung in Anwensenheit von Palladium-auf-Kohle unterwirft.

6. Verfahren zur Herstellung eines 5-(Paradeuterophenyl)-5-phenylhydantoins entsprechend der allgemeinen Formel, dadurch gekennzeichnet, daß man Parabromobenzophenon in Anwesenheit von Dimethylformamid, Ammoniumcarbonat, Kaliumcyanid und Wasser in der Weise umsetzt, daß man 5-(Parabromophenyl)-5-phenylhydantoin erhält und daß man das 5-(Parabromophenyl)-5-phenylhydantoin einer Deuterierung in Anwesenheit von Palladium-auf-Kohle unterwirft.

7. Zusammensetzung gemäß einem der Ansprüche 1 bis 3, dadurch gekennzeichnet, daß sie in Form einer Einzeldosis vorliegt.

8. Zusammensetzung gemäß Anspruch 7, dadurch gekennzeichnet, daß sie in Form einer Pastille, einer Tablette, einer Kapsel, eines Dragees oder einer Lösung vorliegt.

9. Verfahren zur Herstellung einer psychotropen pharmazeutischen Zusammensetzung gemäß einem der Ansprüche 1 bis 3 und 7 und 8, dadurch gekennzeichnet, daß man ein deuteriertes Diphenylhydantoin der allgemeinen Formel mit einem Trägerstoff und/oder festen oder flüssigen, inerten, nicht-toxischen, in der pharmazeutischen Galenik benutzten Exzipientien vermischt und man die Mischung in eine für die Verabreichung geeignete Form bringt wie Tablette, Pastille, Kapsel, Dragee oder trinkbaren oder injizierbaren Lösungen und andere.

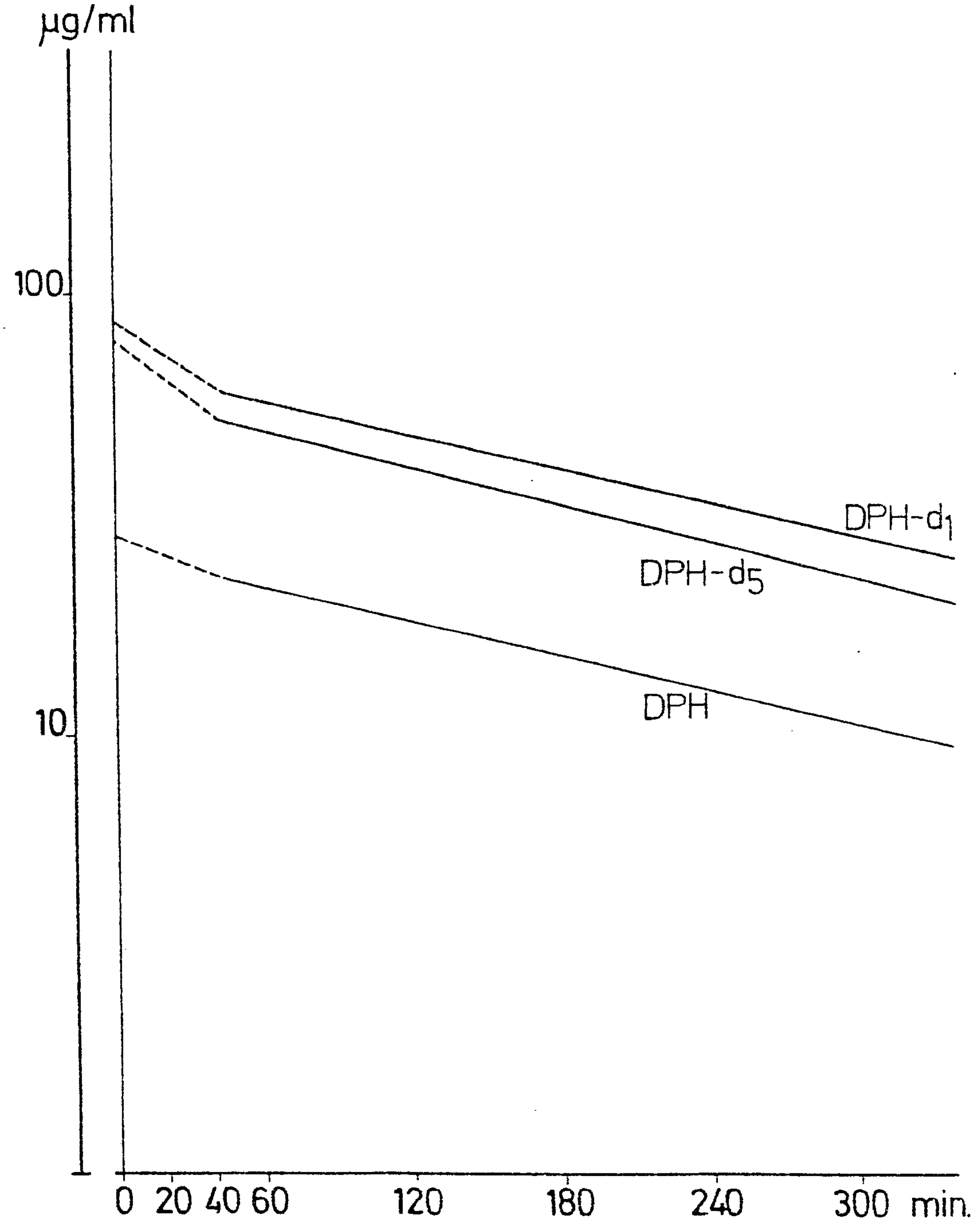
µg/ml
100
10
DPH-d1
DPH-d5
DPH
0 20 40 60 120 180 240 300 min.